# EUROPEAN PATENT APPLICATION

(11) **EP 4 446 721 A1**
(43) Date of publication of application: **16.10.2024**
(21) Application number: 22919963.3
(22) Date of filing: 06.12.2022
(51) Int. Cl.: G01N 1/14

(54) **SAMPLE COLLECTION APPARATUS AND SAMPLE COLLECTION METHOD**

(30) Priority: 13.01.2022 CN 202210036630
(71) Applicant: Assure Tech. (Hangzhou) Co., Ltd., Hangzhou, Zhejiang 310000 (CN)
(72) Inventor: YAO, Lei, Hangzhou, Zhejiang 310000 (CN); LING, Shisheng, Hangzhou, Zhejiang 310000 (CN)
(74) Representative: Wang, Bo
(86) International application number: PCT/CN2022/136774
(87) International publication number: WO 2023/134330

(57) **Abstract**

The embodiments of the present disclosure provide a device for sample collection and a method for sample collection. The device for sample collection comprises a collection part, a detection part, and a connection part configured to connect the collection part and the detection part. The collection part includes a core body. The connection part includes a hollow structure. At least a portion of the core body is disposed in the hollow structure. The core body has hydrophilicity. The device for sample collection is provided with the core body with strong hydrophilicity in the collection part, the core body has a large hydrophilic surface, and the core body absorbs the sample, so that a collected sample volume and efficiency of collection can be improved. In addition, the core body has a maintenance effect on the sample, achieving an unlimited collection angle.

## Description

### CROSS REFERENCE TO RELATED APPLICATIONS

This application claims priority to Chinese Patent Application No. 202210036630.9, filed on January 13, 2022, the contents of each of which are incorporated herein by reference.

### TECHNICAL FIELD

The present disclosure relates to the field of medical devices, and in particular, to a device for sample collection and a method for sample collection.

### BACKGROUND

In the field of sample collection and detection, for liquid samples such as blood and saliva, conventional devices for sample collection mainly absorb biological samples using the siphon effect. Such devices for sample collection have a small collection volume and a limited collection angle, resulting in low detection accuracy and inconvenient operation. Therefore, it is desirable to provide a device for sample collection with a large collection volume and an unlimited collection angle.

### SUMMARY

One of the embodiments of the present disclosure provides a device for sample collection. The device for sample collection may include: a collection part, a detection part, and a connection part configured to connect the collection part and the detection part. The collection part may include a core body. The connection part may include a hollow structure. At least a portion of the core body may be disposed in the hollow structure. The core body may have hydrophilicity.

In some embodiments, the core body may include a hydrophilic fiber bundle structure. Voids for a sample to pass through may be disposed in the fiber bundle structure.

In some embodiments, the fiber bundle structure may be absorbent to red blood cells in the sample.

In some embodiments, the connection part may include a collection end. The collection end may be away from the detection part. The collection end may be provided with a notch. A portion of a surface of the core body may be exposed from the notch.

In some embodiments, the hollow structure may include a first channel and a second channel. The first channel and the second channel may be interconnected and communicated with the connection part. An inner diameter of the first channel may be greater than an inner diameter of the second channel.

In some embodiments, the core body may be disposed in the first channel. An outer diameter of the core body may be greater than the inner diameter of the second channel.

In some embodiments, at least one support structure may be disposed on an inner wall of the first channel. The at least one support structure may protrude from the inner wall of the first channel. The at least one support structure may abut against a peripheral side of the core body.

In some embodiments, the detection part may include a detection cavity. The voids of the core body may be communicated with the detection cavity through the hollow structure.

In some embodiments, the detection part may include a detection element disposed in the detection cavity.

In some embodiments, the detection element may include an adsorption section and a detection section arranged in sequence. The adsorption section may be configured to adsorb red blood cells in the sample. The detection section may be configured to detect the sample.

In some embodiments, the detection element may include a plurality of detection pieces distributed in a radial direction of the detection cavity. Detection objects of the plurality of detection pieces may be different.

In some embodiments, an operation port may be disposed at one end of the detection cavity away from the connection part. The detection element may be placed into and/or taken out of the detection cavity through the operation port. The operation port may be provided with a sealing cover. The sealing cover may be detachably connected with the operation port.

In some embodiments, the detection part may include a bracket configured to support the detection element. The bracket may be connected with an inner wall of the detection cavity.

In some embodiments, the detection part may include a buffer unit disposed between the connection part and the detection cavity. The buffer unit may be configured to reduce a flow velocity of the sample flowing to the detection cavity.

In some embodiments, the detection part may include a connection end connected with the connection part. The connection end may be provided with a limiting structure. The connection part may include a clamping structure. The clamping structure may be connected with the limiting structure in a clamping manner to limit separation of the collection part from the detection part.

In some embodiments, the connection part may include a stop structure. The stop structure may be disposed around a peripheral side of the connection part and extend in a radial direction of the connection part. A diameter of the stop structure may be greater than an inner diameter of the connection end.

In some embodiments, the device for sample collection may further include a buffer agent pre-storage part. The buffer agent pre-storage part may include a buffer agent, a pre-storage cavity, and a sealing sheet. The pre-storage cavity may be configured to store the buffer agent. The sealing sheet may be configured to seal the pre-storage cavity.

In some embodiments, the buffer agent pre-storage part may further include a guide cavity connected with the pre-storage cavity. The sealing sheet may be disposed between the pre-storage cavity and the guide cavity.

In some embodiments, the collection part and the buffer agent pre-storage part may be separately arranged. The collection part may enter the buffer agent pre-storage part through the guide cavity.

One of the embodiments of the present disclosure provides a method for sample collection. The method may include: enabling a collection end of a connection part of a device for sample collection to contact a sample to cause a core body to absorb and store the sample, the core body being disposed in the connection part and having hydrophilicity; inserting the connection part into a buffer agent pre-storage part of the device for sample collection to cause the core body to be inserted into a buffer agent in the buffer agent pre-storage part; and flushing the sample from the core body to a detection part of the device for sample collection using the buffer agent to obtain a detection result.

In some embodiments, the method may further include: connecting the connection part and the detection part.

In some embodiments, the method may further include: placing a detection element in a detection cavity of the detection part; and sealing the detection cavity with a sealing cover.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1A is a front view illustrating an exemplary device for sample collection according to some embodiments of the present disclosure;
FIG. 1B is a side view illustrating an exemplary device for sample collection according to some embodiments of the present disclosure;
FIG. 2 is a cross-sectional view illustrating an exemplary core body according to some embodiments of the present disclosure;
FIG. 3 is a schematic structural diagram illustrating an exemplary collection part and an exemplary connection part according to some embodiments of the present disclosure;
FIG. 4 is a sectional view illustrating an exemplary connection part according to some embodiments of the present disclosure;
FIG. 5A is a schematic structural diagram illustrating an exemplary device for sample collection according to some embodiments of the present disclosure;
FIG. 5B is a schematic structural diagram illustrating an exemplary device for sample collection from another perspective according to some embodiments of the present disclosure;
FIG. 6 is a schematic structural diagram illustrating an exemplary buffer agent pre-storage part according to some embodiments of the present disclosure; and
FIG. 7 is a flowchart illustrating an exemplary process for sample collection according to some embodiments of the present disclosure.

### DETAILED DESCRIPTION

Exemplary embodiments or implementations will be described in detail herein, examples of which are shown in the accompanying drawings. When the following description refers to the drawings, unless otherwise indicated, the same numbers in different drawings represent the same or similar elements. The implementations described in the following exemplary embodiments do not represent all implementations consistent with the present disclosure. Instead, they are merely examples of devices and methods consistent with some aspects of the present disclosure as detailed in the appended claims.

The terms used in the present disclosure are for the purpose of describing specific embodiments only and are not intended to limit the present disclosure. The singular forms "a", "said" and "the" used in the present disclosure and the appended claims are also intended to include plural forms, unless the context clearly indicates other meanings.

It should be understood that the words "first", "second" and similar words used in the specification and claims of the present disclosure do not indicate any order, quantity or importance, but are only used to distinguish different components. Similarly, words such as "one" or "an" do not indicate a quantitative limitation, but rather indicate the presence of at least one. Unless otherwise indicated, words such as "front", "rear", "lower", and/or "upper" are merely for ease of description and are not limited to one position or one spatial orientation. Words such as "include" or "comprise" and similar words mean that the elements or objects appearing before "include" or "comprise" include the elements or objects listed after "include" or "comprises" and their equivalents, and do not exclude other elements or objects.

The device for sample collection of one or more embodiments of the present disclosure may be configured to collect various biological samples or chemical samples, including but not limited to liquid samples such as blood, saliva, urine, chemical solutions, enzyme solutions, drug solutions, etc. The device for sample collection may be further configured to detect the collected samples to provide a reference for disease diagnosis, immune detection, drug testing, etc. In the embodiments of the present disclosure, an exemplary application scenario of the device for sample collection may include collecting and testing blood. The blood may come from reserved blood in a blood collection tube or a blood bag, or from human blood directly obtained by a blood collection needle, such as fingertip blood collection, etc.

In some embodiments, the device for sample collection may be a collection device provided with a siphon tube, which absorbs a sample using the siphon effect of the siphon tube to transfer the sample to a detection cavity for detection. When the device for sample collection is used for collection, due to a limited gravitational force of the siphon tube, a collected sample volume may be small and collection efficiency may be low. In addition, since the sample is affected by gravity, it is usually necessary to place the siphon tube horizontally or at a small angle (e.g., an angle within a range of 0°-10°) to increase the collected sample volume, resulting in a limited collection angle and an inconvenient operation. The device for sample collection of one or more embodiments of the present disclosure may increase the sample collection volume and efficiency by providing a highly hydrophilic core body in a collection part to absorb the sample. In addition, the core body may have a maintenance effect on the sample, so that the collection angle is not limited.

It should be understood that the application scenarios of the device for sample collection of the present disclosure are merely some examples or embodiments of the present disclosure. For those having ordinary skills in the art, the present disclosure may also be applied to other similar scenarios based on these drawings without any creative effort.

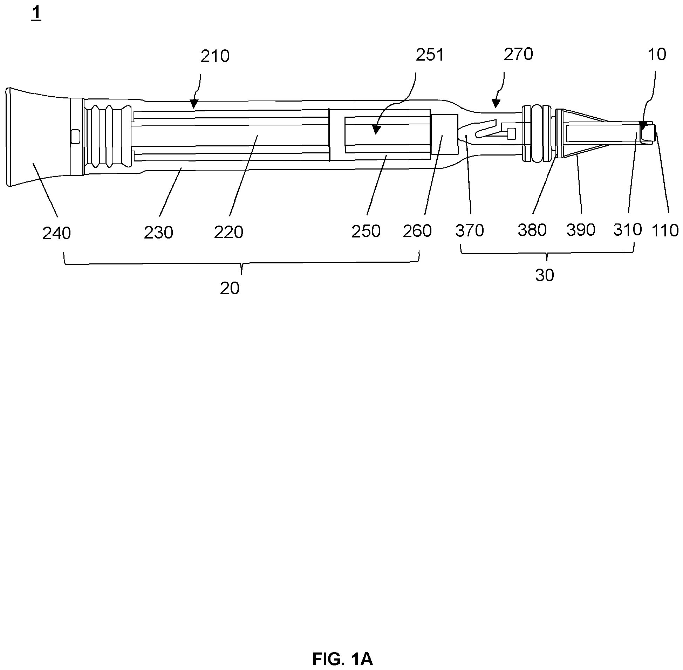

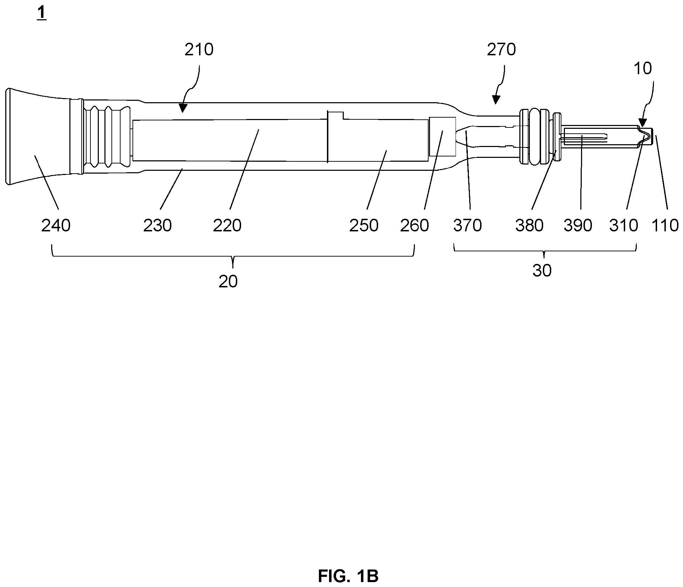

FIG. 1A is a front view illustrating a device 1 for sample collection according to some embodiments of the present disclosure. FIG. 1B is a side view illustrating the device 1 for sample collection according to some embodiments of the present disclosure.

Referring to FIG. 1A and FIG. 1B, the embodiments of the present disclosure provide the device 1 for sample collection. The device 1 for sample collection may include a collection part 10, a detection part 20, and a connection part 30 configured to connect the collection part 10 and the detection part 20. The collection part 10 may be configured to collect a sample. The sample may be a collection object such as blood, saliva, urine, a chemical solution, an enzyme solution, etc. The detection part 20 may be configured to detect various indicators of the sample. In some embodiments, the indicators may include but are not limited to a medical indicator, a biological indicator, a chemical indicator, etc. For example, the sample may be a blood sample, and the detection part 20 may detect indicators such as serum lipids, trace elements, antibiotics, viral antibodies, pH, or the like, of the blood sample. As another example, the sample may be a saliva sample, and the detection part 20 may detect indicators such as enzymes, polysaccharides, alcohol, viral antibodies, or the like, of the saliva sample.

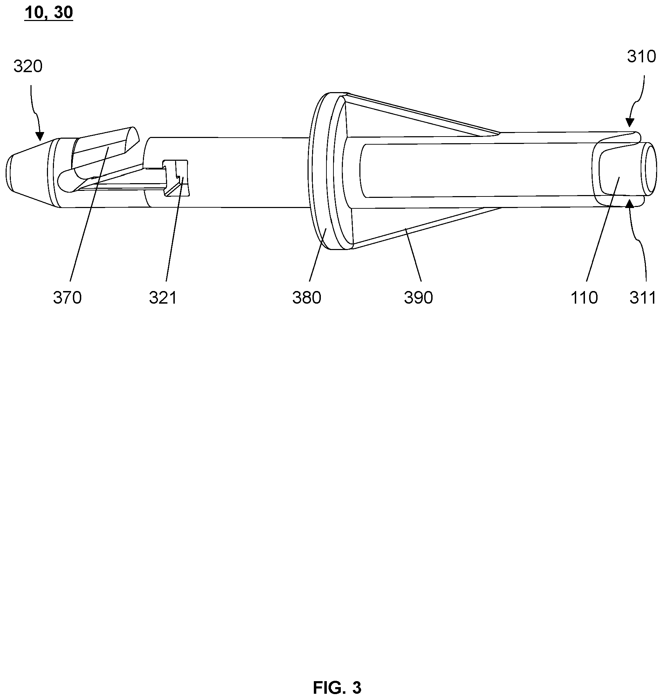

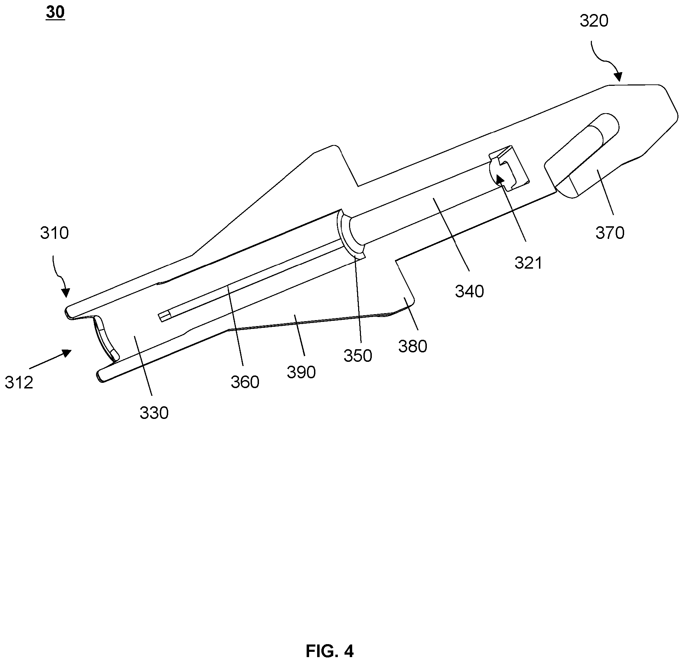

In some embodiments, the collection part 10 may include a core body 110. The connection part 30 may include a hollow structure. At least a portion of the core body 110 may be disposed in the hollow structure of the connection part 30, i.e., the hollow structure of the connection part 30 may accommodate and fix the core body 110. The at least a portion of the core body 110 refers to the entire core body 110 or a portion of the core body 110. In some embodiments, the entire core body 110 may be disposed in the hollow structure of the connection part 30. In some embodiments, the portion of the core body 110 may be disposed in the hollow structure of the connection part 30, and another portion of the core body 110 may be exposed outside the hollow structure of the connection part 30. In some embodiments, the core body 110 may be configured to absorb the sample. In some embodiments, the core body 110 may be further configured to store the sample. More descriptions regarding the collection part 10 may be found in FIG. 3 and FIG. 4 and related descriptions thereof.

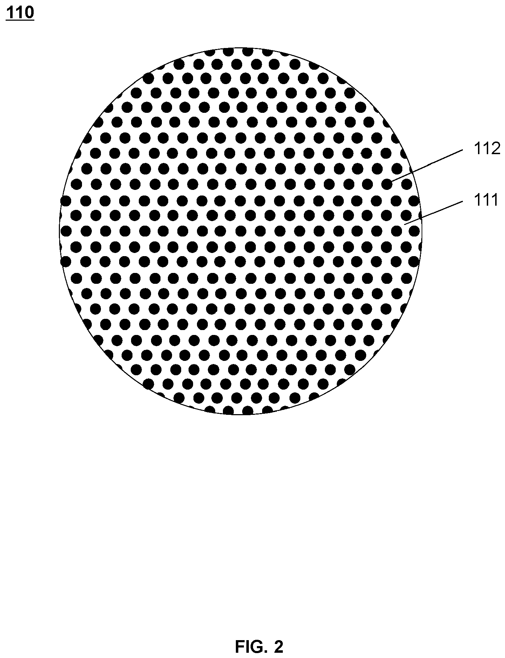

In some embodiments, the core body 110 may have hydrophilicity. Hydrophilicity refers to a property of being able to attract water molecules. The hydrophilic core body 110 may have a great liquid surface tension for the water molecules, so that the water molecules can be absorbed and retained on the core body 110. That is, the core body 110 may be easily wet by the water in the sample. In some embodiments, the hydrophilicity of the core body 110 may include hydrophilicity of an outer surface of the core body 110, and may also include hydrophilicity of an interior of the core body 110. In some embodiments, the hydrophilicity of the core body 110 may be obtained by a special process treatment. In some embodiments, the hydrophilicity of the core body 110 may also be obtained by a property of a material. In some embodiments, the hydrophilicity of the core body 110 may also be obtained by designing an internal structure of the core body 110. The attraction of the core body 110 to the water molecules can be enhanced by the hydrophilicity of the core body 110. Under the action of the attraction, the water molecules quickly diffuse from a wet region of the core body 110 to a dry region of the core body 110, which is manifested as a process of the sample gradually soaking the core body 110. The hydrophilicity of the core body 110 may increase a volume of absorbed sample, meeting the detection requirements of the detection part 20. In addition, the attraction of the core body 110 to the water molecules can increase the speed of absorbing water molecules, improving a sample collection speed. Moreover, after the core body 110 absorbs the sample, the sample stored on the core body 110 may have strong stability and may be less affected by gravity by utilizing the hydrophilicity of the core body 110, which can not only prevent the sample from flowing back, but also allow the collection part 10 to absorb the sample at any angle in space, thereby making the operation of sample collection simpler and more casual. More descriptions regarding the core body 110 may be found in FIG. 2 and related descriptions thereof.

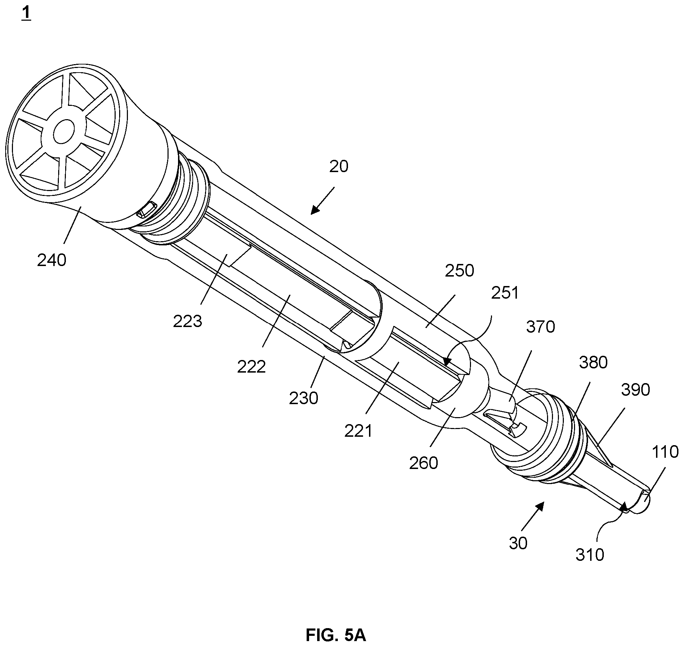

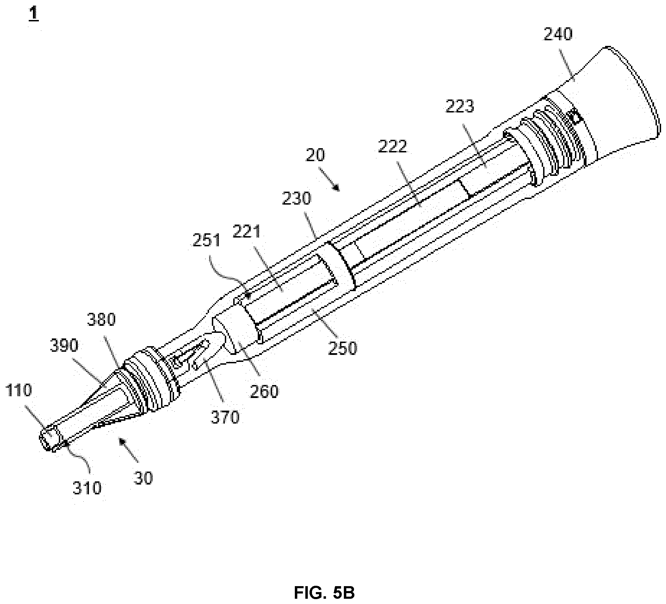

In some embodiments, the detection part 20 may be connected with the collection part 10 through the connection part 30. In some embodiments, the connection part 30 may be detachably connected with the detection part 20, and a connection manner may include but is not limited to snap-fit connection, threaded connection, interference fitting, etc. In some embodiments, the connection part 30 may be non-detachably connected with the detection part 20, and a connection manner may include but is not limited to integrated molding, adhesive connection, etc. In some embodiments, the detection part 20 may include a detection cavity 210 for accommodating the sample when the sample is detected. More descriptions regarding the detection part 20 and the detection cavity 210 may be found in FIG. 5A and FIG. 5B and related descriptions thereof.

FIG. 2 is a cross-sectional view illustrating the core body 110 according to some embodiments of the present disclosure. The arrangement of a fiber bundle structure and the arrangement of voids 111 in FIG. 2 are only for reference, and the arrangement and the density of the fiber bundle structure are not limited in FIG. 2.

Referring to FIG. 2, in some embodiments, the core body 110 may include a hydrophilic fiber bundle structure. The fiber bundle structure refers to a structure in which a large number of single fibers 112 are bonded to each other and solidified into a bundle. A material of the fiber bundle structure may include a material with good biocompatibility and not easy to damage tissue cells in the sample, such as polyester fiber, dacron, medical cotton, etc. Such material may reduce a damage of the material of the core body 110 to the sample, improving the stability of the absorbed sample. Due to the good biocompatibility of the material, a situation that substances in tissue cells affect a detection result caused by rupture of cell membranes of the tissue cells can be prevented. In some embodiments, the fiber bundle structure may be treated by chemical or physical approaches to enhance biocompatibility and other properties of the fiber bundle structure. For example, almost all the fibers 112 in the treated fiber bundle structure may extend in an axial direction of the core body 110. As another example, the treated fiber bundle structure may have a cross-section with equal density, etc.

In some embodiments, the core body 110 may be modified to possess hydrophilicity. In some embodiments, the modification refers to giving the core body 110 new properties, such as hydrophilicity, while maintaining the original properties of the core body 110 or the material of the core body 110. In some embodiments, the single fibers 112 of the core body 110 may be subjected to a surface treatment or cross-sectional treatment to possess hydrophilicity. For example, one or more micro-concaves and convexities may be processed on surfaces of the single fibers 112; as another example, cross sections of the single fibers 112 may be treated into an L shape or a C shape, which can increase surface areas of the single fibers 112, thereby improving hydrophilicity. In some embodiments, hydrophilicity includes attraction to water molecules. In some embodiments, the attraction to water molecules may be enhanced by adding different numbers and types of hydrophilic groups to the core body 110, thereby possessing hydrophilicity. For example, hydrophilicity is enhanced by adding hydrophilic group components such as amino, hydroxyl, carboxyl, amide groups, or the like, to the surfaces or structures of the single fibers 112. In some embodiments, hydrophilicity also includes an ability to maintain the water molecules, which is also referred to as a water locking ability. In some embodiments, hydrophilic groups and hydrophobic groups may be added to the material of the core body 110 simultaneously to form a cross-linked mesh polymer structure. Due to the hydration of the water molecules by the hydrophilic groups, the attraction of the core body 110 to the water molecules can be improved. The hydrophobic groups can make the water molecules entering the mesh lose activity due to polarity, so that the water molecules are maintained inside the material of the core body 110, and the water locking ability of the core body 110 is improved. By providing the hydrophilic material with the cross-linked mesh polymer structure, the water absorption capacity of the core body 110 may reach more than 100 times the weight of the core body 110, greatly improving the attraction and the water locking ability of the core body 110 to the water.

In some embodiments, referring to FIG. 2, after the core body 110 is modified, not only its surface has high hydrophilicity, but also each of the single fibers 112 in the fiber bundle structure has hydrophilicity, so that a total hydrophilic surface area of the entire core body 110 may be increased. The total hydrophilic surface area may be a sum of the surface areas of all the single fibers 112, thereby increasing the attraction of the core body 110 to the water. In some embodiments, the voids 111 for the sample to pass through may be disposed in the fiber bundle structure. The voids 111 may be composed of voids between the single fibers 112, and the sample may pass through the voids 111. The voids 111 inside the fiber bundle structure may increase the hydrophilic area of the core body 110, so that the samples may be more easily absorbed onto the core body 110. In some embodiments, since the core body 110 has the strong attraction and water locking ability to the water molecules, the core body 110 may absorb the sample and store the sample in the voids 111 of the core body 110. The sample may overcome gravity and remain in the core body 110 under the action of the hydrophilicity of the core body 110. In some embodiments, the core body 110 may absorb and store a sample volume of 60 µL-70 µL.

In some embodiments, in the fiber bundle structure, a volume ratio of the voids 111 to the fiber bundle structure may be set in different ranges based on requirements to obtain different effects. For example, the volume ratio of the voids 111 to the fiber bundle structure may be in a range of 1 %-10%. For example, the volume ratio of the voids 111 to the fiber bundle structure may be 3%, 6%, 7%, 8%, 9%, etc.

In some embodiments, the volume ratio of the voids 111 to the fiber bundle structure may be relatively low. For example, the volume ratio of the voids 111 to the fiber bundle structure may be in a range of 1 %-5%, indicating that the fiber bundle structure in the core body 110 may be relatively dense (i.e., the voids between the single fibers 112 may be small), which may produce appropriate resistance to the sample flowing on the core body 110, and slow down a flow velocity of the sample passing through the voids 111, thereby playing a buffering role for the sample. In addition, for the core body 110 with a relatively low volume ratio of the voids 111 to the fiber bundle structure, the performance of maintaining the sample inside may be relatively high, which can prevent the sample from flowing back.

In some embodiments, the volume ratio of the voids 111 to the fiber bundle structure may be relatively high. For example, the volume ratio of the voids 111 to the fiber bundle structure may be in a range of 5%-10%, indicating that the fiber bundle structure in the core body 110 may be relatively sparse (i.e., the voids between single fibers 112 may be relatively large). In this case, flowing resistance to the sample on the core body 10 may be small, which can increase the velocity at which the core body 110 absorbs the sample, allowing the sample to quickly reach the detection cavity 210, thereby improving the detection efficiency.

In the embodiment of the present disclosure, the volume ratio of the voids 111 to the fiber bundle structure may not be too high or too low. If the volume ratio of the voids 111 to the fiber bundle structure is too low, the flow resistance to the sample may be too large, which may reduce the collection efficiency, hinder detection components in the sample (e.g., plasma in a blood sample), and even damage the tissue cells in the sample. If the volume ratio of the voids 111 to the fiber bundle structure is too high, the total hydrophilic surface area of the core body 110 may be reduced too much, reducing the sample volume absorbed by the core body 110. Preferably, the volume ratio of the voids 111 to the fiber bundle structure may be in a range of 5%-6%.

In some embodiments, the volume ratio of the voids 111 to the fiber bundle structure may be in other ranges or set arbitrarily, which is not limited in the present disclosure.

In some embodiments, colored components in a certain sample may interfere with a detection result, so the fiber bundle structure may be treated to have an ability to adsorb colored components or non-detection components in the sample. Taking a blood sample as an example, the red blood cells in the blood sample may affect the detection result, so the fiber bundle structure may have an adsorption property for the red blood cells in the sample. During the collection process, the red blood cells may be retained in the fiber bundle structure, and the serum may continue to flow to the detection cavity 210 for detection.

In some embodiments, the core body 110 may be modified to possess the property of adsorbing the red blood cells. For example, groups such as hemagglutinin capable of adsorbing the red blood cells may be added to the single fibers 112 of the core body 110, so that the red blood cells may bind to the hemagglutinin and other groups to remain in the core body 110.

In some embodiments, the volume ratio of the voids 111 to the fiber bundle structure may be in a range of 5%-10%, so that the core body 110 may have a better property of adsorbing the red blood cells. The volume ratio of the voids 111 to the fiber bundle structure may not be too high or too low. If the volume ratio of the voids 111 to the fiber bundle structure is too high, it means that an amount of the single fibers 112 in the core body 110 is small, and the voids 111 may be large, then an amount of groups such as hemagglutinin may also be small, reducing the adsorption amount of the red blood cells. In addition, if the voids 111 between the single fibers 112 are large, it may easily cause the red blood cells to be flushed to the detection part 20 before completely combined with the groups such as hemagglutinin, reducing the filtering effect of the core body 110 to the red blood cells. If the volume ratio of the voids 111 to the fiber bundle structure is too low, it means that there are more fibers 112 in the core body 110 and the voids 111 may be small. In this case, the red blood cells may easily clog the voids 111, affecting the velocity at which the core body 110 absorbs the sample. In addition, the red blood cells may be easily squeezed and damaged when entering the voids of the core body 110, causing the substances in the red blood cells to be flushed into the detection part 20, and affecting the detection result.

FIG. 3 is a schematic structural diagram illustrating the collection part 10 and the connection part 30 according to some embodiments of the present disclosure. FIG. 4 is a sectional view illustrating the connection part 30 according to some embodiments of the present disclosure.

In some embodiments, referring to FIG. 3, the connection part 30 may be configured as a hollow columnar structure, i.e., the connection part 30 may include a hollow structure. The hollow structure may include but is not limited to a cylindrical shape, a prism shape, etc. In some embodiments, the connection part 30 may include a collection end 310 and an assembly end 320. The collection end 310 may be away from the detection part 20, and may be an end connected with the collection part 10. The assembly end 320 may be an end connected with the detection part 20.

In some embodiments, the collection end 310 may be provided with one or more notches 311. A portion of a surface of the core body 110 may be exposed from the notches 311, thereby increasing an area of the core body 110 exposed outside the collection end 310. When a sample is collected, a contact area between the core body 110 and the sample may be increased by the above setting, thereby improving the sample collection efficiency. A portion of the collection end 310 other than the notches 311 may support the core body 110 to prevent deformation of the core body 110 from affecting the collection effect. In some embodiments, an orthographic projection (referring to a projection in a direction facing the notch) of one of the notches 311 of the collection end 310 may be an arc, a triangle, etc. A count of the notches 311 may be N, wherein N may be a natural number.

In some embodiments, referring to FIG. 4, the hollow structure of the connection part 30 may include a first channel 330 and a second channel 340. The first channel 330 and the second channel 340 may be interconnected and communicated with the connection part 30, so that an inlet 312 allowing the sample to flow in and an outlet 321 allowing the sample to flow out may be formed on the connection part 30. In some embodiments, the inlet 312 of the connection part 30 may be disposed at the collection end 310, and the outlet 321 of the connection part 30 may be disposed at the assembly end 320. In some embodiments, the first channel 330 may be close to the collection end 310. One end of the first channel 330 may form the inlet 312 of the connection part 30, and the other end of the first channel 330 may be communicated with the second channel 340. The second channel 340 may be close to the assembly end 320. One end of the second channel 340 may form the outlet 321 of the connection part 30, and the other end of the second channel 340 may be communicated with the first channel 330. In some embodiments, the first channel 330 may be configured to install the core body 110, and the second channel 340 may be configured as a hollow channel for diversion. In some embodiments, axes of the first channel 330 and the second channel 340 may be straight, curved, etc.

In some embodiments, an inner diameter of the first channel 330 may be greater than an inner diameter of the second channel 340. The inner diameter of the first channel 330 may be relatively larger, to accommodate the core body 110 with a relatively large cross section, increasing the rate of sample collection. The inner diameter of the second channel 340 may be relatively small, which is more likely to produce a capillary phenomenon, so that the sample may flow from the core body 110 toward the outlet 321 under the capillary action.

In some embodiments, the core body 110 may be disposed in the first channel 330, and an outer diameter of the core body 110 may be greater than the inner diameter of the second channel 340. In this case, since the inner diameter of the first channel 330 is greater than the inner diameter of the second channel 340, a step 350 may be formed at a connection between the first channel 330 and the second channel 340, and an end of the core body 110 may abut against the step 350 to limit the core body 110 not to move toward the second channel 340.

In some embodiments, at least one support structure 360 may be disposed on an inner wall of the first channel 330. The at least one support structure 360 may protrude from the inner wall of the first channel 330. The at least one support structure 360 may be configured to support and limit the core body 110. In some embodiments, a protruding height of the at least one support structure 360 from the inner wall of the first channel 330 may be less than a protruding height of the step 350 from the inner wall of the first channel 330, so as to accommodate the core body 110 of which the inner diameter is greater than that of the second channel 340.

In some embodiments, the at least one support structure 360 may be configured as a long protrusion. For example, the long protrusion may extend in an axial direction of the first channel 330, or the long protrusion may extend obliquely relative to a side wall of the first channel 330. In some embodiments, the at least one support structure 360 may be configured as a plurality of protrusion points arranged at intervals. In some embodiments, the at least one support structure 360 may be configured as an annular protrusion. For example, the annular protrusion may be a closed circular ring extending in a circumferential direction, or the annular protrusion may be a spiral ring extending spirally along the inner wall of the first channel 330. It should be noted that the configuration of the at least one support structure 360 in the embodiments of the present disclosure does not constitute a limitation to the embodiments of the present disclosure, and its configuration may be one or a combination of long protrusion, the plurality of protrusion points, the annular protrusion, or other arbitrary protrusion shapes.

In some embodiments, the at least one support structure 360 may include a plurality of support structures 360 abut against a peripheral side of the core body 110. In some embodiments, the arrangement of the at support structures 360 enables the peripheral side of the core body 110 to form a void with the inner wall of the first channel 330. For example, four long protrusions may be arranged at equal intervals in the circumferential direction. The void may be formed between the core body 110 and the inner wall of the first channel 330, reducing a contact area between the core body 110 and the inner wall, thereby reducing the friction resistance of the core body 110 in the process of assembling into the first channel 330, and making it easier for the core body 110 to be inserted into the first channel 330. In addition, the void may be formed between the core body 110 and the inner wall of the first channel 330, so that adhesion and blockage caused by entering of the sample may be avoided. Furthermore, the core body 110 may expand and deform slightly after absorbing the sample, and the void between the core body 110 and the inner wall of the first channel 330 may provide a deformation space for the expansion of the core body 110.

In some embodiments, the connection part 30 may be made of a transparent material, such as transparent plastic, glass, etc., to facilitate observation of the condition of the core body 110 in the first channel 330, such as observation of a degree of sample infiltration into the core body 110, observation of expansion and deformation of the core body 110, etc. In some embodiments, the connection part 30 may be made of a non-transparent material, such as non-transparent plastic, a metallic material, etc.

FIG. 5A is a schematic structural diagram illustrating the device 1 for sample collection according to some embodiments of the present disclosure. FIG. 5B is a schematic structural diagram illustrating the device 1 for sample collection from another perspective according to some embodiments of the present disclosure.

In some embodiments, the detection part 20 may include the detection cavity 210. The voids 111 of the core body 110 may be connected with the detection cavity 210 through a hollow structure of the connection part 30. In this way, after a sample is absorbed into the hollow structure of the connection part 30 by the core body 110, the sample may enter the detection cavity 210 from the hollow structure.

In some embodiments, the detection part 20 may further include a detection element 220 disposed in the detection cavity 210. The detection cavity 210 refers to a hollow cavity capable of accommodating the detection element 220. In some embodiments, the detection cavity 210 may provide a sealed environment for the detection element 220 to prevent substances in an external environment from contaminating the sample and the detection element 220 and affecting a detection result, for example, to prevent external humidity, bacteria, dirt, and other substances from entering the detection cavity 210, ensuring the detection accuracy. In some embodiments, the detection part 20 may include a housing 230. The housing 230 may be configured to define the detection cavity 210 of the detection part 20. In some embodiments, a shape of the housing 230 may include, but is not limited to, a cylindrical tube, a prismatic tube, or an irregular tube, etc. In some embodiments, the housing 230 may be made of a transparent material to facilitate observation of the detection result of the detection element 220. In some embodiments, the detection cavity 210 may be communicated with the hollow structure (e.g., the outlet 321 of the connection part 30) of the connection part 30, so that the voids 111 of the core body 110 may be communicated with the detection cavity 210 through the hollow structure of the connection part 30, thereby realizing transportation of the sample from the collection part 10 to the detection part 20.

In some embodiments, an operation port may be disposed at an end of the detection cavity 210 away from the connection part 30. The detection element 220 may be placed in and/or taken out of the detection cavity 210 from the operation port. In some embodiments, the operation port may be provided with a sealing cover 240. The sealing cover 240 may be detachably connected with the operation port. A manner of detachable connection may include but is not limited to threaded connection, snap connection, or the like, to open or close the operation port. In some embodiments, a socket may be provided on a surface of the sealing cover 240 disposed inside the detection cavity 210. The socket may be configured to fix and install the detection element 220.

In some embodiments, the detection element 220 refers to an element for detecting the sample and displaying a detection result. For example, in detection of a blood sample, a biological enzyme capable of biologically or chemically reacting with the blood sample may be disposed on the detection element 220. When the blood sample reaches the detection element 220, the biological enzyme may react with the blood sample to display a color signal. Detection personnel may determine whether there is a detection substance in the blood sample based on the color signal.

In some embodiments, the detection element 220 may include an adsorption section 221 and a detection section 222 sequentially arranged in an axial direction of the detection cavity 210. In some embodiments, the adsorption section 221 may be disposed at an end close to the connection part 30. After the core body 110 in the connection part 30 collects the sample, most of non-detection components or colored components in the sample may be retained in the core body 110, but a small amount of non-detection components or colored components may reach the detection cavity 210, and the adsorption section 221 may adsorb the non-detection components or colored components reaching the detection cavity 210 again. For example, for the blood sample, the red blood cells in the blood interfere with the color display of the detection element 220. In this case, the core body 110 may be configured to adsorb and retain a large number of red blood cells, and the adsorption section 221 may also be configured to adsorb the red blood cells in the sample, so that even if a small amount of red blood cells enters the detection cavity 210, the small amount of red blood cells may be retained in the adsorption section 221. Through the cooperation of the core body 110 and the adsorption section 221, the non-detection components or the colored components in the sample can be avoided from entering the detection section 222 to the greatest extent, thereby improving the detection accuracy of the detection element 220.

In some embodiments, the detection section 222 may be configured to detect the sample. In some embodiments, the detection section 222 may be provided with substances capable of reacting with the sample, such as a chemical reagent, a reaction enzyme, etc. The substances may cover an entire region or a local region of the detection section 222, which is not limited in the present disclosure. In some embodiments, the detection element may be configured to detect serum lipids, trace elements, antibiotics, pH, etc., which is not limited in the embodiments of the present disclosure.

In some embodiments, the detection element may include a plurality of (two or more) detection pieces. The plurality of detection pieces may detect one or more detection objects of the sample and display detection results. In some embodiments, the plurality of detection pieces may be arranged in the axial direction of the detection cavity 210. In some embodiments, when a detection object of the sample needs to be detected, one of the plurality of detection pieces may be set in the detection cavity 210. An indicator may be set on the detection piece. The indicator may be composed of a substance capable of reacting with the sample. The indicator may form a linear, a two-dimensional graphic, a text, a number or other symbol, which is not limited in the embodiments of the present disclosure. In some embodiments, the indicator of each of plurality of detection pieces may be an indicator line, and different indicator lines may display different colors after encountering different components in the sample to obtain the detection results.

In some embodiments, when a plurality of (two or more) detection objects of the sample need to be detected, one detection piece may be set in the test cavity 210. A plurality of indicators may be set on the detection piece. The plurality of indicators may detect different detection objects, so that a plurality of detection results may be obtained using one sample and one detection piece, thereby improving the detection efficiency. In some embodiments, a count of the indicator lines may be 1-3.

In some embodiments, when the plurality of detection objects of the sample need to be detected, the plurality of detection pieces may also be set in the detection cavity 210. In some embodiments, the detection element 220 may include the plurality of detection pieces distributed in a radial direction of the detection cavity 210. The plurality of detection pieces may be radially distributed in the detection cavity 210 without overlapping or blocking each other, so as to facilitate the observation of the detection results on the plurality of detection pieces. In some embodiments, on a cross section perpendicular to an axis of the detection cavity 210, each of the plurality of detection pieces may extend from the peripheral side of the detection cavity 210 to the axial position of the detection cavity 210. For example, when there are three detection pieces, the three detection pieces may be distributed in three equal portions in the radial direction of the detection cavity 210, and on the cross section perpendicular to the axis of the detection cavity 210, each detection piece may extend from each of positions of the three equal portions of the peripheral side of the detection cavity 210 to the axial position of the detection cavity 210. In some embodiments, the detection objects of the plurality of detection pieces may be different. For example, one indicator may be set on each of the plurality of detection pieces, and each of the plurality of detection pieces may be configured to detect one detection object of the sample. As another example, the plurality of indicators may be set on each of the plurality of detection pieces, and each of the plurality of detection pieces may be configured to detect the plurality of detection objects of the sample. In this way, as many detection objects as possible may be detected using the limited detection pieces, thereby improving the detection efficiency.

In some embodiments, the detection element 220 may further include a connection section 223. One end of the connection section 223 may be connected with a detection section 222, and the other end of the connection section 223 may extend to an end of the detection cavity 210 away from the connection part 30. In some embodiments, one end of the connection section 223 may be connected with the detection section 222, and the other end of the connection section 223 may be inserted into the socket of the sealing cover 240. In some embodiments, the detection element 220 may also not include the connection section 223, and the adsorption section 221 of the detection element 220 may be fixed by a bracket 250 described below. The detection section 222 may be supported in the detection cavity 210 by the adsorption section 221.

In some embodiments, the adsorption section 221, the detection section 222, and the connection section 223 of the detection element 220 may be integrally formed. In some embodiments, a thickness or a hardness of the adsorption section 221 and the connection section 223 may be appropriately increased to provide a greater support force, so that the adsorption section 221 and the connection section 223 may be fixed in the detection cavity 210.

In some embodiments, the detection part 20 may include the bracket 250 configured to support the detection element 220. In some embodiments, the bracket 250 may be provided with a slot, and the adsorption section 221 of the detection element 220 may be inserted into the slot. In some embodiments, the bracket 250 may be connected with an inner wall of the detection cavity 210, so that the stability of the bracket 250 can be maintained. When the device 1 for sample collection is placed in different orientations, the bracket 250 may remain in the detection cavity 210 without shaking. In some embodiments, the bracket 250 may be connected with the inner wall of the detection cavity 210 in the axial direction of the detection cavity 210.

In some embodiments, the bracket 250 may be a hollow cylinder. One end of the bracket 250 may be provided with the slot, and the other end of the bracket 250 may be provided with an opening. The adsorption section 221 of the detection element 220 may be inserted from the slot into the bracket 250 and extend to the opening. In some embodiments, an observation window 251 may be provided on the bracket 250. An adsorption effect of the sample on the adsorption section 221 may be observed through the observation window 251.

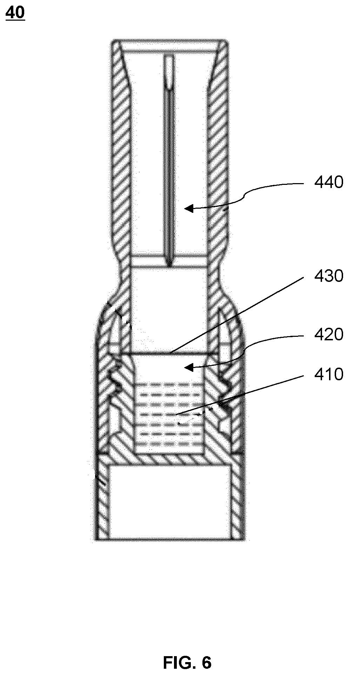

In some embodiments, the detection part 20 may include a buffer unit 260 connected with the detection cavity 210. The buffer unit 260 may be configured to reduce a flow velocity of the sample. After the sample is collected by the core body 110, the sample may be flushed to the detection cavity 210 through a buffer agent 410. Under the action of the flushing force, the sample may flow to the detection cavity 210 at a faster velocity. The buffer unit 260 may slow down the velocity of the sample flowing to the buffer cavity, preventing the sample from splashing to the detection cavity 210 after rushing out of the second channel 340 of the connection part 30. More descriptions regarding the buffer agent 410 may be found in the related descriptions of FIG. 6. In some embodiments, the buffer unit 260 may be configured as a columnar buffer pad, and the buffer unit 260 may be arranged between the connection part 30 and the bracket 250. In some embodiments, the buffer unit 260 may be made of materials such as fiber bundles, polyester fibers 112, cotton, etc. In some embodiments, the detection part 20 may not be provided with the buffer unit 260. By setting the volume ratio of the voids 111 in the core body 110 to the fiber bundle structure, the core body 110 may achieve the effect of sample buffering. The specific setting manner of the volume ratio of the voids 111 to the fiber bundle structure may be found elsewhere in the present disclosure, which is not repeated here.

In some embodiments, when the buffer unit 260 is provided in the detection part 20, the bracket 250 may extend to an end of the buffer unit 260 away from the connection part 30 in the axial direction of the detection cavity 210. In some embodiments, an extension length of the bracket 250 in the detection cavity 210 in the axial direction of the detection cavity 210 may also be set arbitrarily, which is not limited in the embodiments of the present disclosure.

In some embodiments, the detection part 20 may include a connection end 270 connected with the connection part 30. The connection end 270 may be provided with a limiting structure configured to limit displacement of the connection part 30 out of the detection cavity 210. In some embodiments, the limiting structure may be provided on an inner wall of the connection section 223. In some embodiments, the connection part 30 may include a clamping structure 370. The clamping structure 370 may be clamped with the limiting structure to limit separation of the collection part 10 from the detection part 20.

In some embodiments, the limiting structure may be a limiting step (not shown in the figure), and the clamping structure 370 may be a barb having a certain elastic deformation capability. During a process of inserting the connection part 30 into the detection cavity 210, the barb may be radially compressed. After the barb passes through the limiting step, the barb may expand radially under the action of an elastic restoring force and clamp onto the limiting step, thereby limiting the connection part 30 from being separated from the detection cavity 210.

In some embodiments, an inner diameter of the connection end 270 of the detection cavity 210 may be less than inner diameters of other parts of the detection cavity 210 to form the limiting step that matches the clamping structure 370 and make the connection end 270 being more closely matched with the collection part 10. The inner diameters of the other parts of the detection cavity 210 may be greater than the inner diameter of the connection end 270, which can increase a volume of the detection cavity 210 and facilitate the accommodation of the detection element 220.

In some embodiments, the connection part 30 may include a stop structure 380. A diameter of the stop structure 380 may be greater than the inner diameter of the connection end 270. When the connection portion 30 is inserted into the connection end 270, the stop structure 380 may abut against an outer side of the inlet 312 of the connection end 270 to form stopping, preventing the connection part 30 from continuing to move into the connection end 270. In some embodiments, the connection part 30 may include the stop structure 380. The stop structure 380 may be disposed around a peripheral side of the connection part 30 and extend in a radial direction of the connection part 30. The stop structure 380 may be an annular flange protruding from the connection part 30, or may be a bump, etc.

In some embodiments, the connection part 30 may be provided with the clamping structure 370 to be clamped with the limiting structure of the connection end 270 to limit the connection part 30 from being separated from the connection end 270. Meanwhile, the connection part 30 may be provided with the stop structure 380 to abut against the inlet 312 of the connection end 270 to limit the connection part 30 from continuing to enter the connection end 270. By the cooperation between the clamping structure 370 and the stop structure 380, the displacement of the connection part 30 in the axial direction of the detection cavity 210 can be limited, so that the connection part 30 may be fixed at the connection end 270.

In some embodiments, a reinforcing rib 390 may be further provided on the connection part 30. The reinforcing rib 390 may be provided in a region where the stop structure 380 intersects with an outer surface of the connection part 30. The reinforcing rib 390 may be configured to strengthen a strength of the stop structure 380 and the connection part 30.

In some embodiments, the sample collected by the device 1 for sample collection may be obtained from a collection object (e.g., a person being detected). Taking fingertip blood collection as an example, a fingertip may be pierced with a blood collection device, and the core body 110 may directly absorb the blood from the fingertip at any angle. Since the core body 110 has strong hydrophilicity, even if the amount of blood sample collected from the fingertip is small, the blood sample can be continuously absorbed into the core body 110. In some embodiments, after the core body 110 absorbs the blood sample, the blood sample may be stored in the core body 110. Since the core body 110 has extremely strong hydrophilicity and a water locking ability, the blood sample may be retained in the core body 110 after entering the core body 110, and may not flow out of the core body 110 due to gravity. In some embodiments, when the device 1 for sample collection is applied to fingertip blood collection, due to the hydrophilicity of the core body 110, the amount of blood sample collected by the device 1 for sample collection may be as high as a range of 60 µL-70 µL.

FIG. 6 is a schematic structural diagram illustrating an exemplary buffer agent pre-storage part according to some embodiments of the present disclosure.

In some embodiments, referring to FIG. 6, the device 1 for sample collection may further include a buffer agent pre-storage part 40. The buffer agent pre-storage part 40 may include a buffer agent 410, a pre-storage cavity 420, and a sealing sheet 430. The buffer agent 410 may be configured to provide power for the sample stored on the core body 110 to flow to the detection cavity 210. The buffer agent 410 may be stored in the pre-storage cavity 420. The sealing sheet 430 may seal the pre-storage cavity 420 to store the buffer agent 410.

In some embodiments, the buffer agent 410 may include, but is not limited to, a phosphate buffer solution, an organic acid buffer solution, a borate buffer solution, and other solutions. The buffer agent 410 may meet the requirements of mixing and slow-release of the sample, and may have little interference with the components in the sample, may not be prone to chemical precipitation, may not affect pH of the sample, etc. The buffer agent 410 is not limited in the embodiments of the present disclosure.

In some embodiments, the pre-storage cavity 420 may be disposed at a bottom of the buffer pre-storage part 40 for storing the buffer agent 410. In some embodiments, the sealing sheet 430 may block a top of the pre-storage cavity 420 and may be configured as a closed storage space with the pre-storage cavity 420, so that when the buffer pre-storage part 40 is placed in any orientation, the buffer agent 410 may not flow out of the pre-storage cavity 420. In some embodiments, the sealing sheet 430 may be made of a material that can be broken when subjected to a certain pressure (e.g., 100 MPa), so that the collection part 10 may pierce the sealing sheet 430 after being inserted into the buffer pre-storage part 40, and absorb the buffer agent 410. In some embodiments, the sealing sheet 430 may be made of a material that is not easy to break, but a connection strength between the sealing sheet 430 and the top of the pre-storage cavity 420 may be weak, and the sealing sheet 430 may be easy to separate from the top of the pre-storage cavity 420 when subjected to a certain pressure (e.g., 100 MPa), so that the sealing sheet 430 may be pressed into the pre-storage cavity 420, and the collection part 10 can absorb the buffer agent 410.

In some embodiments, the buffer agent pre-storage part 40 may further include a guide cavity connected with the pre-storage cavity 420. The collection part 10 may slide into the pre-storage cavity 420 along the guide cavity. In some embodiments, when the collection part 10 is fully inserted into the buffer agent pre-storage part 40, the guide cavity may accommodate a portion of the detection part 20, and the guide cavity may fit at least a portion of an outer wall of the detection part 20. In some embodiments, a guide groove or a guide protrusion may be provided on an inner wall of the guide cavity to guide the collection part 10. In some embodiments, the sealing sheet 430 may be disposed between the pre-storage cavity 420 and the guide cavity.

In some embodiments, the collection part 10 and the buffer agent pre-storage part 40 may be separately arranged. The core body 110 and the connection part 30 may enter the buffer agent pre-storage part 40 through the guide cavity. When the core body 110 and the connection part 30 are guided to the sealing sheet 430 by the guide cavity, the collection part 10 may continue to be pressed down to make the sealing sheet 430 fail, and the core body 110 may enter the pre-storage cavity 420 to absorb the buffer agent 410.

In some embodiments, the collection part 10 may be temporarily stored in the buffer agent pre-storage part 40 to facilitate matching packaging. In this case, the collection part 10 may be only inserted into the guide cavity and may not continue to apply pressure to the sealing sheet 430.

In some embodiments, the device 1 for sample collection may further include a protective cap. The protective cap may form a cylindrical shape with an open end and a closed end. The collection part 10 may be inserted into the protective cap from the open end, and the protective cap may completely cover the collection part 10. In some embodiments, the open end of the protective cap may be detachably engaged with an outer surface of the detection part 20. In some embodiments, the open end of the protective cap may be detachably engaged with the stop structure 380 of the collection part 10.

In some embodiments, a limiting protrusion may be formed on an outer wall of the protective cap, and a limiting groove adapted to the limiting protrusion may be formed on an inner wall of the guide cavity. After the collecting part 10 is inserted into the protective cap, the protective cap may be inserted into the guide cavity. In this case, the limiting protrusion and the limiting groove may cooperate to limit an insertion depth of the protective cap into the guide cavity, thereby preventing the protective cap from contacting the sealing sheet 430, and preventing the sealing sheet 430 from failing.

In some embodiments, after the collection part 10 collects the sample, the collection part 10 may be inserted into the buffer pre-storage part 40, and the collection part 10 may pierce the sealing sheet 430, so that the collection end 310 of the collection part 10 may be inserted into the buffer agent 410. Since a space inside the pre-storage part 40 is reduced in a process that the core body 110 is inserted into the pre-storage part 40, the buffer agent 410 may flow from the voids 111 of the core body 110 to the detection cavity 210, thereby flushing the sample from the core body 110 to the detection cavity 210.

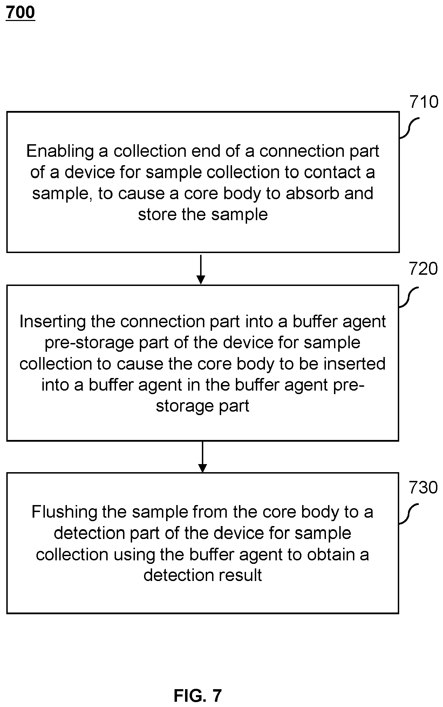

FIG. 7 is a flowchart illustrating an exemplary process for sample collection according to some embodiments of the present disclosure.

Referring to FIG. 7, in some embodiments, the method for sample collection may include a process 700. In some embodiments, the process 700 may be performed by detection personnel and may include the following operations.

In 710, a collection end of a connection part of a device for sample collection may be enabled to contact a sample to cause a core body to absorb and store the sample.

In some embodiments, the connection part 30 of the device 1 for sample collection may have a hollow structure. The core body 110 may be disposed in the hollow structure, and the core body 110 may have hydrophilicity. The descriptions regarding the specific structures of the device 1 for sample collection, the connection part 30, and the core body 110 may be found elsewhere in the present disclosure, which is not repeated here.

In some embodiments, the detection personnel may directly contact a sample to be collected through the collection end 310 of the connection part 30, so that the core body 110 exposed from the notch 311 of the collection end 310 may contact the sample, and the core body 110 may directly absorb the sample at any angle. In some embodiments, after the core body 110 absorbs the sample, the sample may be stored in the core body 110 due to the hydrophilicity of the core body 110.

In some embodiments, to facilitate packaging and transportation, the connection part 30 and the detection part 20 may be placed separately before use. Before the device 1 for sample collection is used for sampling, the connection part 30 may be assembled to the detection part 20. That is, the method for sample collection may further include: connecting the connection part 30 and the detection part 20.

In some embodiments, the detection personnel may insert the assembly end 320 of the connection part 30 into the connection end 270 of the detection cavity 210, so that the clamping structure 370 of the connection part 30 may be clamped with the limiting structure in the detection cavity 210, thereby connecting the connection part 30 to the detection part 20.

In some embodiments, the detection element 220 and the detection part 20 may be placed separately before use. Before the device 1 for sample collection is used for sampling, the detection personnel may select the corresponding detection element 220 according to an item to be detected, and then assemble the detection element 220 into the detection part 20. That is, the device for sample collection may further include: placing the detection element 220 in the detection cavity 210; and sealing the detection cavity 210 with the sealing cover 240.

In some embodiments, the detection personnel may insert the adsorption section 221 of the detection element 220 into the slot of the bracket 250 in the detection cavity 210, and insert the connection section 223 of the detection element 220 into the slot of the sealing cover 240. Then, the detection personnel may place the bracket 250 into the detection cavity 210, so that the bracket 250 may be placed close to the collection part 10. Finally, the detection personnel may seal the detection cavity 210 with the sealing cover 240.

In 720, the connection part may be inserted into a buffer agent pre-storage part of the device for sample collection to cause the core body to be inserted into a buffer agent in the buffer agent pre-storage part.

In some embodiments, the collection part 10 may be inserted into the buffer agent pre-storage part 40 of the device 1 for sample collection. The guide cavity of the buffer agent pre-storage part 40 may guide the collection part 10 to move toward the pre-storage cavity 420 until the collection part 10 reaches the position of the sealing sheet 430.

In some embodiments, the collection part 10 may continue to move toward the pre-storage cavity 420 and generate a pressure on the sealing sheet 430. The sealing sheet 430 may fail under the pressure, allowing the collection part 10 to enter the pre-storage cavity 420.

In 730: the sample may be flushed from the core body to a detection part of the device for sample collection using the buffer agent to obtain a detection result.

In some embodiments, the buffer agent 410 may be stored in the pre-storage cavity 420, and the collection end 310 of the collection part 10 may be immersed in the buffer agent 410. Since a space in the pre-storage part 40 is reduced in a process that the core body 110 is inserted into the pre-storage part 40, the buffer agent 410 may flow from the voids 111 of the core body 110 to the detection cavity 210, thereby flushing the sample from the core body 110 to the detection cavity 210. In some embodiments, the sample may be quickly flushed to the detection cavity 210 of the detection part 20 from the second channel 340 by the buffer agent 410, and under the buffering effect of the buffer unit 260, the sample may slowly flow to the detection element 220 in the detection cavity 210, and reach the detection section 222 after passing through the adsorption section 221 of the detection element 220.

In some embodiments, the detection section 222 may detect components or indicators of the sample and obtain detection results.

The beneficial effects of the embodiments of the present disclosure include but are not limited to the following effects. (1) The collection part includes the core body, and the core body has hydrophilicity capable of increasing the amount of sample absorbed to meet the detection requirements of the detection part, and the core body has a fast absorption velocity, which improves the collection velocity; in addition, with the hydrophilicity of the core body, after the core body absorbs the sample, the sample is stored on the core body with strong stability and is less affected by gravity, which can not only prevent the sample from flowing back, but also allow the collection part to absorb the sample at any angle in space, making the operation of sample collection simpler and more casual. (2) The fiber bundle structure has the voids for the sample to pass through, which can increase the hydrophilic area of the core body and make the sample easier to be absorbed onto the core body. (3) The fiber bundle structure has the adsorption property for the red blood cells in the sample. During the collection process, the red blood cells are retained in the fiber bundle structure, and the serum continues to flow to the detection cavity for detection, thereby preventing the red blood cells from affecting the detection results. (4) The detection element includes an adsorption section and the detection section arranged in sequence. The adsorption section is configured to adsorb the red blood cells in the sample, so that a small amount of red blood cells entering the detection cavity are retained in the adsorption section. With the cooperation of the core and the adsorption section, the detection accuracy of the detection element can be improved. (5) Each of the plurality of detection pieces detects one or more different detection objects, so that a plurality of detection results can be obtained using one sample, which can improve the detection efficiency. (6) The device for sample collection further comprises the protective cap. The protective cap can completely cover the collection part to prevent the protective cap from contacting the sealing sheet, thereby preventing the sealing sheet from failing. It should be noted that different embodiments may produce different beneficial effects. In different embodiments, the beneficial effects that may be produced may be any one or a combination of the above, or any other possible beneficial effects.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

## Claims

1. A device for sample collection, comprising: a collection part, a detection part, and a connection part configured to connect the collection part and the detection part; wherein:
the collection part includes a core body, and the connection part includes a hollow structure, at least a portion of the core body is disposed in the hollow structure, and the core body has hydrophilicity.

2. The device for sample collection of claim 1, wherein the core body includes a hydrophilic fiber bundle structure, and voids for a sample to pass through are disposed in the fiber bundle structure.

3. The device for sample collection of claim 2, wherein the fiber bundle structure is absorbent to red blood cells in the sample.

4. The device for sample collection of claim 1, wherein the connection part includes a collection end, the collection end is away from the detection part, the collection end is provided with a notch, and a portion of a surface of the core body is exposed from the notch.

5. The device for sample collection of claim 1, wherein the hollow structure includes a first channel and a second channel, the first channel and the second channel are interconnected and communicated with the connection part; and an inner diameter of the first channel is greater than an inner diameter of the second channel.

6. The device for sample collection of claim 5, wherein the core body is disposed in the first channel, and an outer diameter of the core body is greater than the inner diameter of the second channel.

7. The device for sample collection of claim 5, wherein at least one support structure is disposed on an inner wall of the first channel, the at least one support structure protrudes from the inner wall of the first channel, and the at least one support structure abuts against a peripheral side of the core body.

8. The device for sample collection of claim 2, wherein the detection part includes a detection cavity, and the voids of the core body are communicated with the detection cavity through the hollow structure.

9. The device for sample collection of claim 8, wherein the detection part includes a detection element disposed in the detection cavity.

10. The device for sample collection of claim 9, wherein:
the detection element includes an adsorption section and a detection section arranged in sequence;
the adsorption section is configured to adsorb red blood cells in the sample; and
the detection section is configured to detect the sample.

11. The device for sample collection of claim 9, wherein the detection element includes a plurality of detection pieces distributed in a radial direction of the detection cavity, and detection objects of the plurality of detection pieces are different.

12. The device for sample collection of claim 9, wherein an operation port is disposed at one end of the detection cavity away from the connection part, the detection element is placed into and/or taken out of the detection cavity through the operation port, the operation port is provided with a sealing cover, and the sealing cover is detachably connected with the operation port.

13. The device for sample collection of claim 10, wherein the detection part includes a bracket configured to support the detection element, and the bracket is connected with an inner wall of the detection cavity.

14. The device for sample collection of claim 8, wherein the detection part includes a buffer unit disposed between the connection part and the detection cavity, and the buffer unit is configured to reduce a flow velocity of the sample flowing to the detection cavity.

15. The device for sample collection of claim 1, wherein:
the detection part includes a connection end connected with the connection part, and the connection end is provided with a limiting structure; and
the connection part includes a clamping structure, and the clamping structure is connected with the limiting structure in a clamping manner to limit separation of the collection part from the detection part.

16. The device for sample collection of claim 15, wherein the connection part includes a stop structure, the stop structure is disposed around a peripheral side of the connection part and extends in a radial direction of the connection part, and a diameter of the stop structure is greater than an inner diameter of the connection end.

17. The device for sample collection of claim 4, further comprising a buffer agent pre-storage part, wherein:
the buffer agent pre-storage part includes a buffer agent, a pre-storage cavity, and a sealing sheet; and
the pre-storage cavity is configured to store the buffer agent, and the sealing sheet is configured to seal the pre-storage cavity.

18. The device for sample collection of claim 17, wherein the buffer agent pre-storage part further includes a guide cavity connected with the pre-storage cavity, and the sealing sheet is disposed between the pre-storage cavity and the guide cavity.

19. The device for sample collection of claim 18, wherein the collection part and the buffer agent pre-storage part are separately arranged, and the collection part enters the buffer agent pre-storage part through the guide cavity.

20. A method for sample collection, comprising:
enabling a collection end of a connection part of a device for sample collection to contact a sample, to cause a core body to absorb and store the sample, wherein the core body is disposed in the connection part and has hydrophilicity;
inserting the connection part into a buffer agent pre-storage part of the device for sample collection, to cause the core body to be inserted into a buffer agent in the buffer agent pre-storage part; and
flushing the sample from the core body to a detection part of the device for sample collection using the buffer agent, to obtain a detection result.

21. The method of claim 20, further comprising:
connecting the connection part and the detection part.

22. The method of claim 21, further comprising:
placing a detection element in a detection cavity of the detection part; and
sealing the detection cavity with a sealing cover.
